# EUROPEAN PATENT SPECIFICATION

(11) **EP 4 041 913 B1**
(45) Date of publication and mention of the grant of the patent: **29.11.2023**
(21) Application number: 20789241.5
(22) Date of filing: 05.10.2020
(51) Int. Cl.: C12Q 1/6806

(54) **NOVEL METHOD**
NEUARTIGES VERFAHREN
NOUVEAU PROCÉDÉ

(30) Priority: 04.10.2019 GB 201914325
(43) Date of publication of application: 17.08.2022
(73) Proprietor: Babraham Institute, Cambridge, Cambridgeshire CB22 3AT (GB)
(72) Inventor: MALYSHEVA, Valeriya, Cambridge Cambridgeshire CB22 3AT (GB); SCHOENFELDER, Stefan, Cambridge Cambridgeshire CB22 3AT (GB); SPIVAKOV, Mikhail, Cambridge Cambridgeshire CB22 3AT (GB); NAGANO, Takashi, Cambridge Cambridgeshire CB22 3AT (GB); FRASER, Peter, Cambridge Cambridgeshire CB22 3AT (GB)
(74) Representative: Sagittarius IP
(86) International application number: PCT/GB2020/052448
(87) International publication number: WO 2021/064430

(56) References cited:
- WO-A1-2014/168575
- WO-A1-2015/033134
- WO-A1-2017/068379
- STEFAN SCHOENFELDER ET AL: "Promoter Capture Hi-C: High-resolution, Genome-wide Profiling of Promoter Interactions", JOURNAL OF VISUALIZED EXPERIMENTS, no. 136, 28 June 2018 (2018-06-28), XP055748772, DOI: 10.3791/57320

## Description

### FIELD OF THE INVENTION

The present invention relates to a method for identifying nucleic acid segments which interact with a target nucleic acid segment or segments as well as kits for performing the method. The invention also relates to a method of identifying one or more interacting nucleic acid segments that are indicative of a particular disease.

### BACKGROUND OF THE INVENTION

Regulatory elements play a central role in an organism's genetic control and have been shown to contribute to health and disease (e.g. in cancer and autoimmune disorders). It has been demonstrated that such regulatory elements (for example, enhancers) can be located at considerable genomic distances (on a linear scale) from their target genes. Approaches for capture of these regulatory elements and their target genes have been developed and are widely applied to study the impact of regulatory landscape dynamics on gene expression and phenotype establishment, as well as to study the role of genetic modifications in disease development. However, when working with low cell numbers, determining which target genes these regulatory elements regulate represents a major challenge.

One of the first methods developed to identify interactions between genomic loci was Chromosome Conformation Capture (3C) technology (Dekker et al. Science (2002) 295: 1306-1311). This involved creating a 3C library by: crosslinking a nuclear composition so that genomic loci that are in close spatial proximity become linked; removing the intervening DNA loop between the crosslink by digestion; and ligating and reversing crosslinking of the interacting regions to generate a 3C library. The library can then be used to detect/identify the frequency of interactions between known sequences. However, this method has a requirement of previous knowledge of the interaction in order to detect the interacting regions of interest. Since then, the technology has been further developed to overcome limitations with the 3C method.

Hi-C is a genome-wide method that does not require any prior knowledge about the interactome of interest. This method uses junction markers to isolate all of the ligated interacting sequences in the cell (see WO 2010/036323 and Lieberman-Aiden *et al.* 2009). Although this provides information on all interactions occurring within the nuclear composition at a particular time point, the resulting libraries are extremely complex which impedes their analysis at a resolution required to identify significant interactions between specific elements, such as promoters and enhancers. To overcome this limitation, the capture Hi-C technique has been developed which involves a capture step to enrich Hi-C libraries for chromosomal interactions comprising, at least at one end, the regions of interest (see WO 2015/033134, Dryden *et al.* 2014 and Schoenfelder *et al.,* 2015). WO 2015/033134 discloses a method and kit for identifying nucleic acid segments which interact with a target nucleic acid segment by use of an isolating nucleic acid molecule. However, this method requires starting with a large number of cells (30-40 million cells), which is impossible when working with rare cell types, cells from the early stages of organism development or patient/biopsy samples.

WO 2014/168575 discloses a targeted chromosome conformation capture method that comprises the steps of: crosslinking of genomic DNA; fragmentation by sonication and biotin marker fill-in; proximity ligation; purifying the biotin-marked fragments; adding sequencing adapters; adding labelled capture probes and selective purification following PCR amplification; and analysing the captured fragments by sequencing using the adapters added after biotin-purification.

WO 2017/068379 discloses a chromatin conformation capture (3C) method comprising the steps of: fragmenting a 3C library produced from a nucleic acid sample; followed by optionally adding sequencing adaptors to the ends of the fragments and/or amplifying the fragments; isolating a subgroup of fragments using a targeting nucleic acid and a binding pair; and optionally sequencing the isolated fragments using any adapters added after fragmentation.

There is therefore a need to provide an improved method for identifying nucleic acid interactions which overcomes the limitations of the currently available methodologies.

### SUMMARY OF THE INVENTION

According to a first aspect of the invention, there is provided a method for identifying nucleic acid segments which interact with a target nucleic acid segment or segments, said method comprising the steps of:
(a) obtaining a nucleic acid composition comprising the target nucleic acid segment or segments;
(b) crosslinking the nucleic acid composition;
(c) fragmenting the crosslinked nucleic acid composition using an endonuclease enzyme;
(d) filling the ends of the fragmented crosslinked nucleic acid segments with one or more nucleotides comprising a covalently linked biotin moiety;
(e) ligating the fragmented nucleic acid segments obtained from step (d) to produce ligated fragments;
(f) performing single step fragmentation and oligonucleotide insertion on the ligated fragments using a recombinase enzyme;
(g) enriching for fragments comprising the biotin moiety of step (d);
(h) enriching for fragments comprising the target nucleic acid segment or segments segments in the presence of sequences which prevent the binding of ligated fragments to other ligated fragments through complementarity of adapter sequences, such as blocker sequences;
(i) sequencing the enriched fragments obtained in step (h) to identify the nucleic acid segments which interact with the target nucleic acid segment or segments.

According to a further aspect of the invention, there is provided a method of identifying one or more interacting nucleic acid segments that are indicative of a particular disease state, comprising:
(a) performing a method as defined herein on a nucleic acid composition obtained from an individual with a particular disease;
(b) quantifying a frequency of interaction between a nucleic acid segment and a target nucleic acid segment or segments; and
(c) comparing the frequency of interaction in the nucleic acid composition from the individual with said disease state with the frequency of interaction in a normal control nuclear composition from a healthy subject, such that a difference in the frequency of interaction in the nucleic acid composition is indicative of a particular disease.

According to a yet further aspect of the invention, there is provided a kit for identifying a nucleic acid segment which interacts with a target nucleic acid segment or segments, comprising buffers and reagents suitable for performing the steps of the methods defined herein in combination with sequences which prevent the binding of ligated fragments to other ligated fragments through complementarity of adapter sequences, such as blocker sequences.

### BRIEF DESCRIPTION OF THE FIGURES

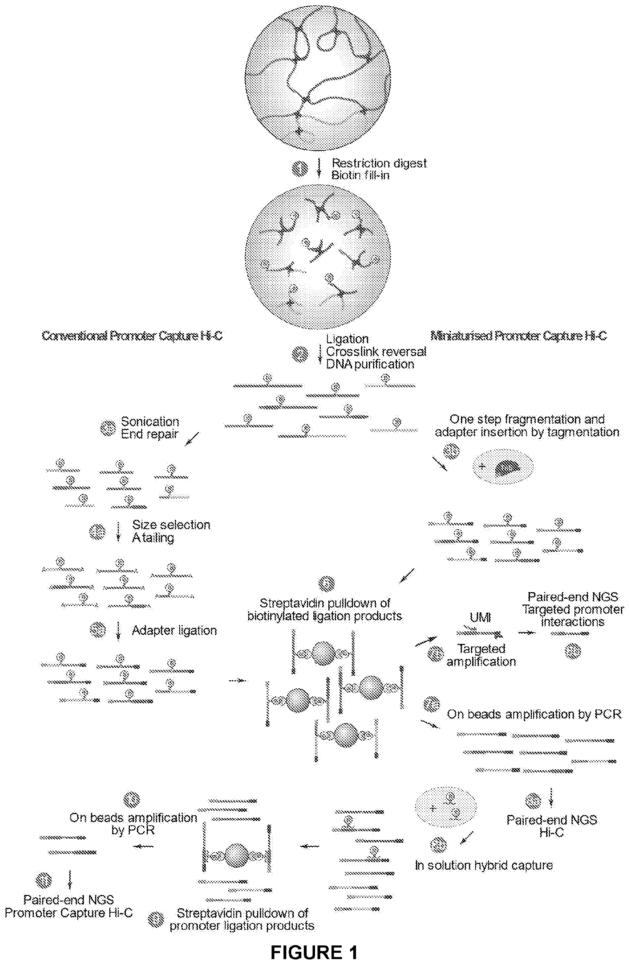

**Figure 1****:** Comparative schematic overview of the conventional protocol compared to miniaturised promoter capture Hi-C protocol presented herein. Abbreviations: Tn5 - recombinase/transposase enzyme, B - biotin, NGS - next-generation sequencing, UMI - unique molecular identifier.

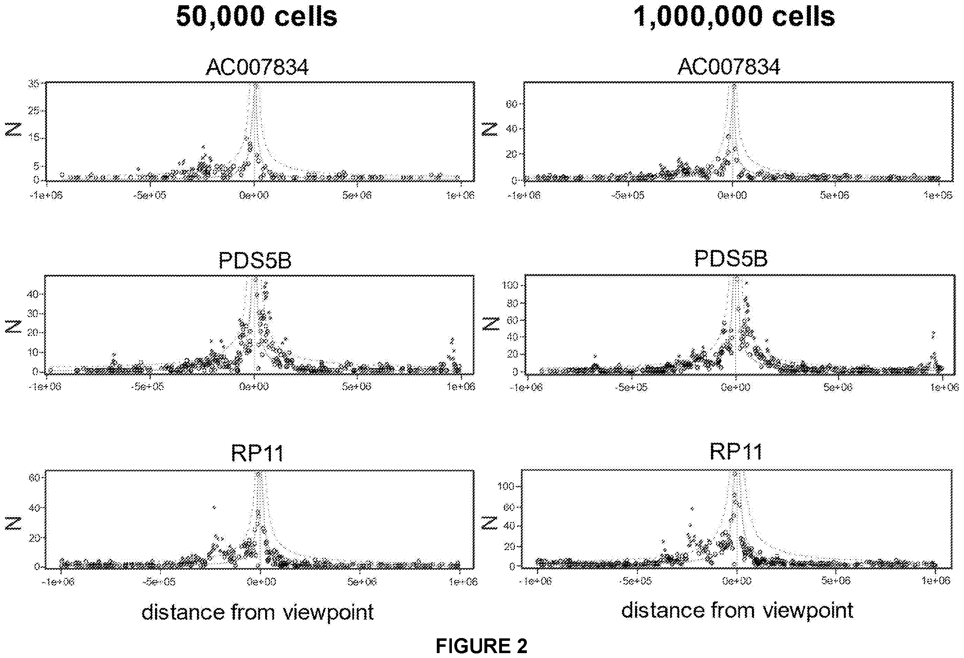

**Figure 2****:** Results obtained using the methods as defined herein. Significant interactions were called using CHiCAGO in test samples - human CD4+ T cells with 50000 cells and 1 million cells starting material (1/600 and 1/30 of the starting material used in a conventional Hi-C protocol, respectively).

### DETAILED DESCRIPTION OF THE INVENTION

According to a first aspect of the invention, there is provided a method for identifying nucleic acid segments which interact with a target nucleic acid segment or segments, said method comprising the steps of:
(a) obtaining a nucleic acid composition comprising the target nucleic acid segment or segments;
(b) crosslinking the nucleic acid composition;
(c) fragmenting the crosslinked nucleic acid composition using an endonuclease enzyme;
(d) filling the ends of the fragmented crosslinked nucleic acid segments with one or more nucleotides comprising a covalently linked biotin moiety;
(e) ligating the fragmented nucleic acid segments obtained from step (d) to produce ligated fragments;
(f) performing single step fragmentation and oligonucleotide insertion on the ligated fragments using a transposase enzyme;
(g) enriching for fragments comprising the biotin moiety of step (d);
(h) enriching fragments comprising the target nucleic acid segment or segments in the presence of sequences which prevent the binding of ligated fragments to other ligated fragments through complementarity of adapter sequences, such as blocker sequences;
(i) sequencing the enriched fragments obtained in step (h) to identify the nucleic acid segments which interact with the target nucleic acid segment or segments.

The method of the present invention provides a means for identifying interacting sequences and nucleic acid segments by using either targeted amplification or isolating nucleic acid molecules to isolate a target nucleic acid segment or segments. Such methods have the advantage of focussing the data on particular interactions within enormously complex libraries. Furthermore, methods comprising targeted amplification or addition of isolating nucleic acid molecules which bind to the target nucleic acid segment or segments can also be used to organise the information into various subsets depending on the type of reagents used for selection or the type of isolating nucleic acid molecules used (e.g. promoters to identify promoter interactions). Detailed information on the chromosomal interactions within a particular group of targets of interest can then be obtained.

The methods of the present invention further provide a single step fragmentation and oligonucleotide insertion using a recombinase enzyme. Such single step fragmentation and oligonucleotide insertion has the advantage of providing a method with significantly fewer overall steps and reduced manipulation of the nucleic acid composition. For example, in particular embodiments of the present method, a single tube may be utilised from obtaining a nucleic acid composition (step (a) as defined herein) to ligating the fragmented nucleic acid segments (step (e) as defined herein) and also from performing single step fragmentation and oligonucleotide insertion (step (f) as defined herein) to enrichment of fragments comprising biotin (step (g) as defined herein). Methods which do not comprise single step fragmentation and oligonucleotide insertion require separate fragmentation by physical or enzymatic means (e.g. sonication or restriction enzyme digestion), end repair of library fragments, addition of dATP at the 3'-end of library fragments, size selection, ligation of oligonucleotide sequences and purification of fragments from unligated oligonucleotides (such as those methods disclosed in WO 2015/033134). Therefore, it will be appreciated that the present invention provides methods for identifying nucleic acid segments which interact with a target nucleic acid segment or segments which are simpler, comprise fewer steps and may comprise shorter time-frames for completion. Thus, the methods of the present invention are faster than conventional protocols and moreover decrease the overall cost of library production. It will also be appreciated that such advantages may lead to reduced loss of nucleic acid composition. Such reduced loss of nucleic acid composition allows for the amount of starting material to be reduced, for example the number of cells from which the nucleic acid composition is obtained, or the increase in resulting nucleic acid composition which is available for subsequent analysis.

Furthermore, whereas previous techniques, such as 4C, allow the capture of genome-wide interactions of one or a few promoters, the methods described herein can capture over 22000 promoters and their interacting genomic loci in a single experiment. Moreover, the present methods yield a significantly more quantitative readout.

Genome-Wide Association Studies (GWAS) have identified thousands of single-nucleotide polymorphisms (SNPs) that are linked to disease. However, many of these SNPs are located at great distances from genes, making it very challenging to predict on which genes they act. Therefore, the present methods provide ways of identifying interacting nucleic acid segments, even if they are located far away from each other within the genome.

References to "nucleic acid segments" as used herein, are equivalent to references to "nucleic acid sequences", and refer to any polymer of nucleotides (i.e. for example, adenine (A), thymidine (T), cytosine (C), guanosine (G), and/or uracil (U)). This polymer may or may not result in a functional genomic fragment or gene. A combination of nucleic acid sequences may ultimately comprise a chromosome. A nucleic acid sequence comprising deoxyribonucleosides is referred to as deoxyribonucleic acid (DNA). A nucleic acid sequence comprising ribonucleosides is referred to as ribonucleic acid (RNA). RNA can be further characterised into several types, such as protein-coding RNA, messenger RNA (mRNA), transfer RNA (tRNA), long non-coding RNA (InRNA), long intergenic non-coding RNA (lincRNA), antisense RNA (asRNA), micro RNA (miRNA), short interfering RNA (siRNA), small nuclear (snRNA) and small nucleolar RNA (snoRNA).

"Single-nucleotide polymorphisms" or "SNPs" are single nucleotide variations (i.e. A, C, G or T) within a genome that differ between members of a biological species or between paired chromosomes.

It will be understood that the term "target nucleic acid segment or segments" refers to the sequence or sequences of interest which are known to the user. Isolating only the ligated fragments which contain the target nucleic acid segment or segments helps to focus the data to identify specific interactions with a particular gene or gene segment of interest. Alternatively, performing targeted amplification to enrich fragments comprising the target nucleic acid segment or segments helps to focus the data to identify specific interactions with a particular gene or gene segment of interest by increasing the proportion of fragments within the composition which comprise the target nucleic acid segment or segments.

References herein to the term "interacts" or "interacting", refer to an association between two elements, for example in the present method, a genomic interaction between a nucleic acid segment and a target nucleic acid segment. The interaction may cause one interacting element to have an effect upon the other, for example, silencing or activating the element it binds to. The interaction may occur between two nucleic acid segments that are located close together or far apart on the linear genome sequence. Thus, in one embodiment, the nucleic acid segment or segments which interact with a target nucleic acid segment or segments are in close proximity to said target nucleic acid segment or segments on the linear genome sequence, for example, are relatively close to each other on the same chromosome. In a further embodiment, the nucleic acid segment or segments which interact with a target nucleic acid segment or segments are located far apart from said target nucleic acid segment or segments on the linear genome sequence, for example, present on a different chromosome or further away if on the same chromosome.

References herein to the term "nucleic acid composition", refers to any composition comprising nucleic acids and protein. The nucleic acids within the nucleic acid composition may be organised into chromosomes, wherein the proteins (i.e. for example, histones) may become associated with the chromosomes having a regulatory function. In one embodiment, the nucleic acid composition comprises a nuclear composition. Such a nuclear composition may typically include a nuclear genome organisation or chromatin.

References to "crosslinking" or "crosslink" as used herein, refer to any stable chemical association between two compounds, such that they may be further processed as a unit. Such stability may be based upon covalent and/or non-covalent bonding (e.g. ionic). For example, nucleic acids and/or proteins may be crosslinked by chemical agents (i.e. for example, a fixative), heat, pressure, change in pH, or radiation, such that they maintain their spatial relationships during routine laboratory procedures (i.e. for example, extracting, washing, centrifugation etc.). Crosslinking as used herein is equivalent to the terms "fixing" or "fixation", which applies to any method or process that immobilises any and all cellular processes. A crosslinked/fixed cell, therefore, accurately maintains the spatial relationships between components within the nucleic acid composition at the time of fixation. Many chemicals are capable of providing fixation, including but not limited to, formaldehyde, formalin, or glutaraldehyde.

References to the term "fragments" as used herein, refers to any nucleic acid sequence that is shorter than the sequence from which it is derived. Fragments can be of any size, ranging from several megabases and/or kilobases to only a few nucleotides long. Fragments are suitably greater than 5 nucleotide bases in length, for example 10, 15, 20, 25, 30, 40, 50, 100, 250, 500, 750, 1000, 2000, 5000 or 10000 nucleotide bases in length. Fragments may be even longer, for example 1, 5, 10, 20, 25, 50, 75, 100, 200, 300, 400 or 500 nucleotide kilobases in length. Methods such as restriction enzyme digestion, sonication, acid incubation, base incubation, microfluidization etc., can all be used to fragment a nucleic acid composition.

In some embodiments, fragmentation (i.e. step (c)) is performed using an endonuclease enzyme. Examples of suitable endonuclease enzymes include, but are not limited to, sequence specific endonucleases, such as restriction enzymes, and non-sequence specific endonucleases, such as MNase or DNase.

Thus, in one embodiment, the endonuclease enzyme is a sequence specific endonuclease, such as a restriction enzyme. The term "restriction enzyme" as used herein, refers to any protein that cleaves nucleic acid at a specific base pair sequence. Cleavage can result in a blunt or sticky end, depending on the type of restriction enzyme chosen. Examples of restriction enzymes include, but are not limited to, Eco Rl, Eco RII, Bam HI, Hind III, Dpn II, Bgl II, Nco I, Taq I, Not I, Hinf I, Sau 3A, Pvu II, Sma I, Hae III, Hga I, Alu I, Eco RV, Kpn I, Pst I, Sac I, Sal I, Sca I, Spe I, Sph I, Stu I, Xba !. In a further embodiment, fragmentation (i.e. step (c)) is performed using a restriction enzyme. In one embodiment, the restriction enzyme is Hind III. In a further embodiment, the restriction enzyme is Dpn II.

In an alternative embodiment, the endonuclease enzyme is a non-sequence specific endonuclease. The term "non-sequence specific endonuclease" as used herein, refers to any protein that cleaves nucleic acid and is not restricted to the sequence of said nucleic acid, for example they may cleave nucleic acid at any region where protein (e.g. nucleosomes and/or transcription factors) is not bound. Examples of non-sequence specific endonucleases are known in the art and include, but are not limited to, DNase, RNase and MNase. MNase is a non-specific endo-exonuclease derived from the bacteria Staphylococcus aureus, which binds and cleaves protein-unbound regions of DNA on chromatin - DNA bound to histones or other chromatin-bound proteins remains undigested. In a yet further embodiment, fragmentation (i.e. step (c)) is performed using a non-sequence specific endonuclease.

In another embodiment, fragmentation (i.e. step (c)) is performed using sonication.

References herein to the term "filling the end(s)" of fragments or of nucleic acid segments, refer to the addition of nucleotides to the 3' end of the crosslinked nucleic acid composition or segments following fragmentation. Such filling comprises the addition of dATP, dCTP, dGTP and/or dTTP nucleotides to the 3' end of the nucleic acid composition or segments. In order to allow the enrichment of nucleic acid fragments or segments which have been ligated and thus contain a ligation junction, one or more of the nucleotides used for filling as described herein may comprise a covalently linked biotin moiety. Thus, in one embodiment, filling the ends of fragmented crosslinked nucleic acid segments comprises the addition of a biotin moiety to the ends of the crosslinked nucleic acid fragments. In a further embodiment, filling the ends of the fragmented crosslinked nucleic acid segments comprises "marking" the ends of the crosslinked nucleic acid fragments with a "junction marker". Such "marking" of the ends or addition of a biotin moiety to the ends of the crosslinked nucleic acid fragments allows for the subsequent selection, or enrichment, of nucleic acid fragments and/or segments which have been ligated according to step (e) and the methods as defined herein.

The junction marker allows ligated fragments to be purified prior to enrichment step (h), therefore ensuring that only ligated sequences are enriched, rather than non-ligated (i.e. non-interacting) fragments.

In certain embodiments, the junction marker comprises a labelled nucleotide linker (i.e. a nucleotide comprising a covalently linked biotin moiety). In a further embodiment, the junction marker comprises biotin. In one embodiment, the junction marker may comprise a modified nucleotide. In one embodiment, the junction marker may comprise an oligonucleotide linker sequence.

References herein to the terms "ligated" or "ligating", refer to any linkage of two nucleic acid segments usually comprising a phosphodiester bond. The linkage is normally facilitated by the presence of a catalytic enzyme (i.e. for example, a ligase such as T4 DNA ligase) in the presence of co-factor reagents and an energy source (i.e. for example, adenosine triphosphate (ATP)). In the methods described herein, the fragments of two nucleic acid segments that have been crosslinked are ligated together in order to produce a single ligated fragment.

In one embodiment, ligation of fragmented nucleic acid segments to produce ligated fragments (i.e. step (e)) utilises in-nucleus ligation. Thus, in certain embodiments, ligation of fragmented nucleic acid segments is performed by in-nucleus ligation. Such in-nucleus ligation has the advantage that small volumes of reagents may be used, leading to reduced loss of nucleic acid composition, and thus may also allow for the amount of starting material to be reduced. For example, the number of cells from which the nucleic acid composition is obtained may be reduced, or the resulting nucleic acid composition which is available for subsequent analysis may be increased.

References herein to "single step fragmentation and oligonucleotide insertion", refer to the fragmentation of ligated fragments and insertion of oligonucleotide sequences in a single step. Such methods utilise a recombinase enzyme which binds to the oligonucleotide sequences and inserts these onto the fragments. This process is also known as "tagmentation". Therefore, in one embodiment, single step fragmentation and oligonucleotide insertion comprises tagmentation.

Advantages of single step fragmentation and oligonucleotide ligation, further to those mentioned above, include that any binding pair element (such as biotin) which has been incorporated into the nucleic acid composition does not need to be removed from unligated fragments, no size selection of ligated fragments need be performed, enzymatic fragmentation by a recombinase removes the need for end repair as no sonication has been performed, and the addition of A-tails need not be performed. Furthermore, the insertion of oligonucleotide and/or adapter sequences, which may include barcode sequences and/or a unique molecular identifier, is performed concurrently with fragmentation. Such barcode sequences or unique molecular identifier may allow for the identification of a particular nucleic acid composition in subsequent analysis and processing and allow for multiple nucleic acid compositions to be combined in subsequent steps, whilst retaining the ability to identify and analyse individual nucleic acid compositions. Thus, in one embodiment, the oligonucleotide sequence is an "adapter" sequence which allows for or enables subsequent library preparation and sequencing of the adapter-containing nucleic acid fragments. In a further embodiment, the adapter comprises a barcode sequence and/or unique molecular identifier.

In a yet further embodiment, single step fragmentation and oligonucleotide insertion comprises inserting barcode sequences into the ligated fragments. In one embodiment, paired end adapter sequences comprise barcode sequences and/or a unique molecular identifier.

A yet further advantage of methods of the invention utilising single step fragmentation and oligonucleotide ligation (e.g. tagmentation) as presented herein is the obtaining of a significantly enriched library of fragments comprising the target nucleic acid segment or segments compared to previously published protocols. For example, enrichment values of between at least 5-fold and 20-fold or between at least 5-fold and 80-fold compared to libraries produced according to previously known or conventional Hi-C protocols may be generated. In one embodiment, a library at least 5-fold, at least 10-fold, at least 15-fold or at least 20-fold enriched may be generated according the methods defined herein, compared to a library generated according to conventional Hi-C protocols. In a further embodiment, a library at least 10-fold, at least 11-fold, at least 12-fold, at least 13-fold, at least 14-fold or at least 15-fold enriched may be generated according the methods defined herein. In a yet further embodiment, a library at least 50-fold, at least 55-fold or at least 60-fold enriched may be generated according the methods defined herein. It will be appreciated that any enrichment value for a library which is obtained when performing the methods as defined herein, compared to a library generated according to conventional Hi-C protocols, can be dependent on the identity of the endonuclease enzyme used for fragmenting the crosslinked nucleic acid composition. For example, when the restriction enzyme Hind III is used, an enrichment value of up to 20-fold may be obtained. Alternatively, when the restriction enzyme Dpn II is used, an enrichment value of up to 80-fold may be obtained.

The term "paired end adapters" as used herein, refers to any primer pair set that allows automated high throughput sequencing to read from both ends. For example, such high throughput sequencing devices that are compatible with these adapters include, but are not limited to Solexa (Illumina), the 454 System, and/or the ABI SOLiD. For example, the method may include using universal primers in conjunction with poly-A tails.

Recombinase enzymes suitable for use in the present methods will be appreciated to include any enzyme capable of removing (or cutting) and inserting sequence into an oligonucleotide or nucleic acid fragment. Examples of such recombinase enzymes include retroviral integrase and transposase enzymes such as MuA, Tn5, Tn7 and Tc1/mariner-type transposases. Thus, in one embodiment of the present method, the recombinase enzyme is a retroviral integrase. In a further embodiment, the recombinase enzyme is a transposase enzyme, such as Tn5 transposase. In order for the recombinase, integrase or transposase enzyme to be active in the method presented herein, the enzyme may be mutated to overcome the naturally occurring low level of activity of such enzymes. Thus, in a yet further embodiment, the recombinase enzyme is a mutant transposase, such as a hyperactive transposase. Such a hyperactive transposase may be a mutant Tn5 transposase. In one embodiment, the recombinase is Tn5 transposase, such as hyperactive Tn5 transposase.

Tn5 transposase is a member of the RNase superfamily of recombinase proteins which includes retroviral integrases and catalyses the movement of a portion of nucleic acid, known as a transposon, to another part of or another genome by a so called "cut and paste" mechanism. Recombinases, such as transposase enzymes, and transposon elements can be found in certain bacteria and are involved in the acquisition of antibiotic resistance.

Transposase enzymes are commonly inactive and mutations in either the active site or elsewhere in the protein can lead to the generation of a hyperactive enzyme. Methods of producing Tn5 transposase enzyme are known in the art (Picelli et al. (2014) Genome Research 24:2033-2040). However, these methods may be further adapted by utilising oligonucleotide sequences, such as adapter sequences, when purifying the Tn5 transposase enzyme.

Oligonucleotide sequences, such as adapter sequences, used when purifying the recombinase enzyme (e.g. the Tn5 transposase) incorporate with the enzyme and are subsequently inserted by said recombinase into a nucleic acid fragment or segment. Such sequences may be diverse in their sequence and comprise additional elements which enable further processing of the nucleic acid fragment or segment into which they are inserted. For example, oligonucleotides incorporated with a purified recombinase enzyme may comprise an adapter sequence for sequencing and/or a barcode sequence. It will be appreciated, however, that all such oligonucleotides comprise a transposon sequence or element which allows for incorporation with the enzyme. Examples of transposon sequences or elements include the Tn5 transposase-compatible Mosaic End (ME) sequence and sequences which are sterically compatible with the binding pocket of a recombinase and/or transposase enzyme.

Thus, according to one embodiment, the recombinase enzyme of the method comprises Mosaic End Double-Stranded (MEDS) oligonucleotides, which comprise a half of paired end adapter sequences. In a further embodiment, the recombinase enzyme comprises paired end adapter sequences for sequencing. In yet further embodiments, the transposase enzyme may comprise oligonucleotides comprising paired end adapter sequences for sequencing which additionally comprise barcode sequences. In further embodiments, the oligonucleotide sequences are selected from: SEQ ID NO: 1, SEQ ID NO: 2 and/or SEQ ID NO: 3 as defined herein. In an alternative embodiment, the oligonucleotide sequences comprise any sequence that enables subsequent library preparation and sequencing. Such sequences will be appreciated to enable the amplification and isolation of nucleic acid segments as well as the binding of said nucleic acid segments for analysis of sequence by high-throughout or next generation sequencing. Examples of next generation sequencing platforms include: Roche 454 (i.e. Roche 454 GS FLX), Applied Biosystems' SOLiD system (i.e. SOLiDv4), Illumina's GAllx, HiSeq 2000 and MiSeq sequencers, Life Technologies' Ion Torrent semiconductor-based sequencing instruments, Pacific Biosciences' PacBio RS and Oxford Nanopore's MiniON.

References herein to "enriching" or "enrichment", refer to any isolation of nucleic acid segments or increase in the proportion of nucleic acid segments of interest or target nucleic acid segments relative to other nucleic acid segments within the nucleic composition. It will be appreciated that such references include the terms "isolating", "isolation", "separating", "removing", "purifying" and the like. For example, the enrichment or isolation of nucleic acid segments of interest or target nucleic acid segments may comprise positive methods, such as the "pulling out" of nucleic acid segments of interest or target nucleic acid segments, or may comprise negative methods, such as the exclusion of nucleic acid segments which are not of interest or which do not comprise a target nucleic acid segment. Alternatively, enriching or isolating may comprise the selective or targeted amplification of nucleic acid segments of interest or target nucleic acid segments. Such selective or targeted amplification of nucleic acid segments of interest or target nucleic acid segments will increase the proportion of such segments in the nucleic acid composition (i.e. enrich said segments).

In one embodiment, said enrichment step (h) comprises the step of performing targeted amplification to enrich fragments comprising the target nucleic acid segment or segments.

In an alternative embodiment, said enrichment step (h) comprises the steps of:
(i) addition of isolating nucleic acid molecules which bind to the target nucleic acid segment or segments, wherein said isolating nucleic acid molecules are labelled with a first half of a binding pair; and
(ii) isolating fragments which contain the target nucleic acid segment or segments bound to the isolating nucleic acid molecules by using the second half of said binding pair,
in order to enrich fragments comprising the target nucleic acid segment or segments. In certain embodiments steps (i) and (ii) above may be performed sequentially, that is step (i) is performed and followed by step (ii). In further embodiments, steps (i) and (ii) above may be performed concurrently.

Thus, enrichment step (h) of the present method comprises the enrichment of nucleic acid fragments or segments of interest or target nucleic acid segments comprising a particular target segment or sequence.

References herein to "targeted amplification" refer to amplification using methods which preferentially amplify particular nucleic acid segments of interest or target nucleic acid segments. Such targeted amplification may utilise particular primer sequences which are complementary to target nucleic acid segments or sequences present within target nucleic acid segments (e.g. a promoter or silencer sequence). Thus, in one embodiment, the primer sequences are complementary to a promoter sequence. In another embodiment, the primer sequences are complementary to a sequence comprising a SNP. Primer sequences utilised in the methods presented herein may comprise additional elements involved in subsequent processing or analysis of amplified nucleic acid segments. For example, primer sequences may comprise adapter sequences for sequencing as described herein or a unique molecular identifier useful for identification of a nucleic acid segment or group of segments (e.g. those derived from a particular sample). Alternatively or additionally, targeted amplification may utilise particular conditions which may favour the amplification of target nucleic acid segments or fragments comprising target nucleic acid segments. It will be appreciated that amplification may be performed by any method known in the art, such as polymerase chain reaction (PCR). It will be further appreciated that targeted amplification as described herein may comprise amplification of nucleic acid segments in solution or on a support moiety, such as a bead, used for enrichment. Elongation of primer sequences may also be performed prior to amplification, such that amplification on a support moiety may additionally comprise a step of elongation of primer sequences prior to the amplification of said elongated sequences and nucleic acid segments.

References herein to an "isolating nucleic acid molecule" refer to a molecule formed of nucleic acids that is configured to bind to the target nucleic acid segment or segments. For example, the isolating nucleic acid molecule may contain the complementary sequence to the target nucleic acid segment or segments which will then form interactions with the nucleotide bases of the target nucleic acid segment or segments (i.e. to form base pairs (bp)). It will be understood that the isolating nucleic acid molecule, for example biotinylated RNA, does not need to contain the entire complementary sequence of the target nucleic acid segment or segments in order to form complementary interactions and isolate it from the nucleic acid composition. The isolating nucleic acid molecule may be at least 10 nucleotide bases long, for example, at least 20, 30, 40, 50, 60, 70, 80, 90, 100, 130, 150, 170, 200, 300, 400, 500, 750, 1000, 2000, 3000, 4000 or 5000 nucleotide bases long.

In one embodiment, the addition of isolating nucleic acid molecules which bind to the target nucleic acid segment or segments is performed at between 65°C and 72°C. In a particular embodiment, the addition of isolating nucleic acid molecules is performed at 65°C. Thus, in a further embodiment, step (i) of enrichment step (h) above is performed at between 65°C and 72°C, such as at 65°C. In another embodiment, isolating fragments which contain the target nucleic acid segment or segments bound to the isolating nucleic acid molecules using the second half of said binding pair is performed at between 68°C and 72°C. In a particular embodiment, isolating fragments using the second half of the binding pair is performed at 68°C. Thus, in a yet further embodiment, step (ii) of enrichment step (h) is performed at between 68°C and 72°C, such as at 68°C.

In one embodiment, the isolating nucleic acid molecules are added in the presence of blocking or blocker sequences. Such blocker sequences prevent the binding of ligated fragments comprising adapter sequences to other ligated fragments comprising adapter sequences through any complementarity in the sequence of the adapter sequences. Thus, such blocker sequences prevent binding of fragments which do not comprise the target nucleic acid segment or segments to fragments which do comprise the target nucleic acid segment or segments. In certain embodiments, the blocker sequences are added to the ligated fragments prior to the addition of isolating nucleic acid molecules. In alternative embodiments, the blocker sequences are added to the ligated fragments concurrently, or together with, the isolating nucleic acid molecules. It will therefore be appreciated that, in one embodiment, the blocker sequences comprise any sequence compatible with the adapter sequences ligated to fragments, such as a sequence complementary to the particular adapter sequence. In a further embodiment, the blocker sequences comprise any sequence compatible with, such as complementary to, the MEDS oligonucleotides comprising half of paired end adapter sequences. In some embodiments, the blocker sequences are selected from: SEQ ID NO: 10, SEQ ID NO: 11, SEQ ID NO: 12, SEQ ID NO: 13, SEQ ID NO: 14, SEQ ID NO: 15, SEQ ID NO: 16 and/or SEQ ID NO: 17 as defined herein.

Additionally, enrichment step (h) of the present methods may be performed according to methods and utilising reagents known in the art. For example, wherein enrichment step (h) comprises isolating nucleic acid molecules which bind to the target nucleic acid segment or segments as described herein, the method or steps of the method may be performed in the presence of a buffer with high concentrations of divalent cation salt, such as between 100mM and 600mM. The salt may be present at a molar ratio of between 2.5:1 and 60:1. A volume-excluding/thickening agent may also be present, for example in a concentration of between 0.002% and 0.1%. Additionally or alternatively, said method or steps of the method may comprise incubating the nucleic acid composition in the presence of a buffer. Incubation may be for a period of 8 hours or less, optionally at two different temperatures, wherein the two different temperatures are cycled between 2 and 100 times. Examples of such buffers and methods are described in US 9,587,268. It will thus be appreciated that, according to wherein enrichment step (h) is performed according to certain embodiments disclosed herein, enrichment comprising isolating nucleic acid molecules is more rapid than when using conventional reagents and reaction methods.

References herein to a "binding pair" refer to at least two moieties (i.e. a first half and a second half) that specifically recognise each other in order to form an attachment. Suitable binding pairs include, for example, biotin and avidin or biotin and derivatives of avidin such as streptavidin and neutravidin.

References herein to "labelling" or "labelled" refer to the process of distinguishing a target by attaching a marker, wherein the marker comprises a specific moiety having a unique affinity for a ligand (i.e. an affinity tag). For example, the label may serve to selectively purify the isolating nucleic acid sequence (i.e. for example, by affinity chromatography). Such a label may include, but is not limited to, a biotin label, a histidine label (i.e. 6His), or a FLAG label.

In one embodiment, the isolating nucleic acid molecules comprise biotin. In a further embodiment, the isolating nucleic acid molecules are labelled with biotin. In a yet further embodiment, the isolating nucleic acid molecules are labelled with a histidine label or a FLAG label. Thus, according to certain embodiments, the binding pair may comprise a label (such as a histidine or FLAG label) and an antibody.

In one embodiment, the target nucleic acid segment or segments are selected from promoters, enhancers, silencers or insulators. In a further embodiment, the target nucleic acid segment or segments are promoters. In a further alternative embodiment, the target nucleic acid segment or segments are insulators.

References herein to the terms "promoter" and "promoters", refer to nucleic acid sequences which facilitates the initiation of transcription of an operably linked coding region. Promoters are sometimes referred to as "transcription initiation regions". Regulatory elements often interact with promoters in order to activate or inhibit transcription.

The present inventors have used the method of the invention to identify thousands of promoter interactions, with ten to twenty interactions occurring per promoter. The method described herein has identified some interactions to be cell specific, or to be associated with different disease states. A wide range of separation distances between interacting nucleic acid segments has also been identified - most interactions are within 100 kilobases, but some can extend to 2 megabases and beyond. Interestingly, the method has also been used to show that both active and inactive genes form interactions.

Nucleic acid segments that are identified to interact with promoters are candidates to be regulatory elements that are required for proper genetic control. Their disruption may alter transcriptional output and contribute to disease, therefore linking these elements to their target genes could provide potential new drug targets for new therapies.

Identifying which regulatory elements interact with promoters is crucial to understanding genetic interactions. The present method also provides a snapshot look at the interactions within the nucleic acid composition at a particular point in time, therefore it is envisaged that the method could be performed over a series of time points or developmental states or experimental conditions to build a picture of the changes of interactions within the nucleic acid composition of a cell.

It will be understood that in one embodiment the target nucleic acid segment interacts with a nucleic acid segment which comprises a regulatory element. In a further embodiment, the regulatory element comprises an enhancer, silencer or insulator.

The term "regulatory gene" as used herein, refers to any nucleic acid sequence encoding a protein, wherein the protein binds to the same or a different nucleic acid sequence thereby modulating the transcription rate or otherwise affecting the expression level of the same or a different nucleic acid sequence. The term "regulatory element" as used herein, refers to any nucleic acid sequence that affects the activity status of another genomic element. For example, various regulatory elements may include, but are not limited to, enhancers, activators, repressors, insulators, promoters or silencers.

In one embodiment, the target nucleic acid molecule is a genomic site identified through chromatin immunoprecipitation (ChIP) sequencing. ChIP sequencing experiments analyse protein-DNA interactions by crosslinking protein-DNA complexes within a nucleic acid composition. The protein-DNA complex is then isolated (by immunoprecipitation) prior to sequencing the genomic region to which the protein is bound.

It will be envisaged that in some embodiments, the nucleic acid segment is located on the same chromosome as the target nucleic acid segment. Alternatively, the nucleic acid segment is located on a different chromosome to the target nucleic acid segment.

The method may be used to identify a long range interaction, a short range interaction or a close neighbour interaction. The term "long range interaction" as used herein, refers to the detection of interacting nucleic acid segments that are far apart within the linear genome sequence. This type of interaction may identify two genomic regions that are, for instance, located on different arms of the same chromosome, or located on different chromosomes.

The term "short range interaction" as used herein, refers to the detection of interacting nucleic acid segments that are located relatively close to each other within the genome. The term "close neighbour interaction" as used herein, refers to the detection of interacting nucleic acid segments that are very close to each other in the linear genome and, for instance, part of the same gene.

SNPs have been shown by the present inventors to be positioned more often in an interacting nucleic acid segment than would be expected by chance, therefore the method of the present invention can be used to identify which SNPs interact with, and are therefore likely to regulate, specific genes.

Thus, from the disclosures presented herein, it will be appreciated that the present methods may by used to identify any nucleic acid interactions, in particular DNA-DNA interactions within a nucleic acid composition.

In one embodiment, the isolating nucleic acid molecule is obtained from bacterial artificial chromosomes (BACs), fosmids or cosmids. In a further embodiment, the isolating nucleic acid molecule is obtained from bacterial artificial chromosomes (BACs).

In one embodiment, the isolating nucleic acid molecule is DNA, cDNA or RNA. In a further embodiment, the isolating nucleic acid molecule is RNA.

The isolating nucleic acid molecule may be employed in a suitable method, such as solution hybridization selection (see WO 2009/099602). In this method a set of 'bait' sequences is generated to form a hybridization mixture that can be used to isolate a sub group of target nucleic acids from a sample (i.e. 'pond').

In one embodiment, the first half of the binding pair comprises biotin and the second half of the binding pair comprises streptavidin.

In one embodiment, the method additionally comprises reversing the cross-linking prior to step (f). It will be understood that there are several ways known in the art to reverse crosslinks and it will depend upon the way in which the crosslinks are originally formed. For example, crosslinks may be reversed by subjecting the crosslinked nucleic acid composition to high heat, such as above 50°C, 55°C, 60°C, 65°C, 70°C, 75°C, 80°C, 85°C, or greater. Furthermore, the crosslinked nucleic acid composition may need to be subjected to high heat for longer than 1 hour, for example, at least 5 hours, 6 hours, 7 hours, 8 hours, 9 hours, 10 hours or 12 hours or longer. In one embodiment, reversing the cross-linking prior to step (f) comprises incubating the crosslinked nucleic acid composition at 65°C for at least 8 hours (i.e. overnight) in the presence of Proteinase K.

In one embodiment, the method additionally comprises purifying the nucleic acid composition to remove any fragments which do not contain the junction marker prior to step (f).

References herein to "purifying", may refer to a nucleic acid composition that has been subjected to treatment (i.e. for example, fractionation) to remove various other components, and which composition substantially retains its expressed biological activity. Where the term "substantially purified" is used, this designation will refer to a composition in which the nucleic acid forms the major component of the composition, such as constituting about 50%, about 60%, about 70%, about 80%, about 90%, about 95% or more of the composition (i.e. for example, weight/weight (w/w), volume/volume (v/v) and/or weight/volume (w/v)).

In one embodiment, the method additionally comprises amplifying the isolated target ligated fragments prior to step (i). In a further embodiment, the amplifying is performed by polymerase chain reaction (PCR).

In one embodiment, the nucleic acid composition is derived from a mammalian cell nucleus. In a further embodiment, the mammalian cell nucleus may be a human cell nucleus. Many human cells are available in the art for use in the method described herein, for example GM12878 (a human lymphoblastoid cell line) or CD34+ (human ex *vivo* haematopoietic progenitors).

It will be appreciated that the method described herein finds utility in a range of organisms, not just humans. For example, the present method may also be used to identify genomic interactions in plants and animals.

Therefore, in an alternative embodiment, the nucleic acid composition is derived from a non-human cell nucleus. In one embodiment, the non-human cell is selected from the group including, but not limited to, plants, yeast, mice, cows, pigs, horses, dogs, cats, goats, or sheep. In one embodiment, the non-human cell nucleus is a mouse cell nucleus or a plant cell nucleus.

It will be appreciated from the advantages of the invention as mentioned herein, that the present methods provide for a reduced loss of nucleic acid composition during the herein mentioned steps. Such reduced loss of nucleic acid composition may allow for the amount of starting material to be reduced, for example the number of cells from which the nucleic acid composition is obtained. Thus, in one embodiment, the nucleic acid composition may be derived from a smaller number of cells than previous promoter capture or conformation capture techniques. In a further embodiment, the nucleic acid composition is derived from 1 million or fewer cells, 0.5 million or fewer cells, 0.2 million or fewer cells, 50000 or fewer cells or 10000 or fewer cells. In a yet further embodiment, the nucleic acid composition is derived from 1 million cells, 0.5 million cells, 0.2 million cells, 50000 cells or 10000 cells. In certain embodiments, the nucleic acid composition is derived from 1 million cells, 50000 cells or 10000 cells.

In one embodiment, the method as defined herein comprises the steps of:
(i) crosslinking a nucleic acid composition comprising the target nucleic acid segment or segments;
(ii) fragmenting the crosslinked nucleic acid composition;
(iii) marking the ends of the fragments with biotin;
(iv) ligating the fragmented nucleic acid segments to produce ligated fragments;
(v) reversing the crosslinking;
(vi) performing single step fragmentation and adapter insertion on the ligated fragments using a transposase enzyme;
(vii) pulldown of ligated fragments with streptavidin;
(viii) performing targeted amplification of fragments comprising the target nucleic acid segment or segments; and
(ix) sequencing to identify the nucleic acid segments which interact with the target nucleic acid segment or segments.

In another embodiment, the method as defined herein comprises the steps of:
(i) crosslinking a nucleic acid composition comprising the target nucleic acid segment or segments;
(ii) fragmenting the crosslinked nucleic acid composition;
(iii) marking the ends of the fragments with biotin;
(iv) ligating the fragmented nucleic acid segments to produce ligated fragments;
(v) reversing the crosslinking;
(vi) performing single step fragmentation and adapter insertion on the ligated fragments using a transposase enzyme;
(vii) pulldown of fragments with streptavidin and amplification using PCR;
(viii) promoter capture by addition of isolating nucleic acid molecules which bind to the target nucleic acid segment or segments in the presence of sequences which prevent the binding of ligated fragments to other ligated fragments through complementarity of adapter sequences, such as blocker sequences, wherein said isolating nucleic acid molecules are labelled with a first half of a binding pair;
(ix) isolating ligated fragments which contain the target nucleic acid segment or segments bound to the isolating nucleic acid molecules by using the second half of the binding pair;
(x) amplification using PCR; and
(xi) sequencing to identify the nucleic acid segments which interact with the target nucleic acid segment or segments.

According to a further aspect of the invention, there is provided a method of identifying one or more interacting nucleic acid segments that are indicative of a particular disease state comprising:
a) performing the method defined herein on a nucleic acid composition obtained from an individual with a particular disease state;
b) quantifying a frequency of interaction between a nucleic acid segment and a target nucleic acid segment or segments;
c) comparing the frequency of interaction in the nucleic acid composition from the individual with said disease state with the frequency of interaction in a normal control nucleic acid composition from a healthy subject, such that a difference in the frequency of interaction in the nucleic acid composition is indicative of a particular disease.

References to "frequency of interaction" or "interaction frequency" as used herein, refer to the number of times a specific interaction occurs within a nucleic acid composition (i.e. sample). In some instances, a lower frequency of interaction in the nucleic acid composition, compared to a normal control nucleic acid composition from a healthy subject, is indicative of a particular disease state (i.e. because the nucleic acid segments are interacting less frequently). Alternatively, a higher frequency of interaction in the nucleic acid composition, compared to a normal control nucleic acid composition from a healthy subject, is indicative of a particular disease state (i.e. because the nucleic acid segments are interacting more frequently). In some instances, the difference will be represented by at least a 0.5-fold difference, such as a 1-fold, 1.5-fold, 2-fold, 2.5-fold, 3-fold, 4-fold, 5-fold, 7-fold or 10-fold difference.

In one aspect of the invention, the frequency of interaction may be used to determine the spatial proximity of two different nucleic acid segments. As the interaction frequency increases, the probability increases that the two genomic regions are physically proximal to one another in 3D nuclear space. Conversely, as the interaction frequency decreases, the probability decreases that the two genomic regions are physically proximal to one another in 3D nuclear space.

Quantifying can be performed by any method suitable to calculate the frequency of interaction in a nucleic acid composition from a patient or a purification or extract of a nucleic acid composition sample or a dilution thereof. For example, high throughput sequencing results can also enable examination of the frequency of a particular interaction. In methods of the invention, quantifying may be performed by measuring the concentration of the target nucleic acid segment or ligation products in the sample or samples. The nucleic acid composition may be obtained from cells in biological samples that may include cerebrospinal fluid (CSF), whole blood, blood serum, plasma, or an extract or purification therefrom, or dilution thereof. In one embodiment, the biological sample may be cerebrospinal fluid (CSF), whole blood, blood serum or plasma. Biological samples also include tissue homogenates, tissue sections and biopsy specimens from a live subject, or taken post-mortem. The samples can be prepared, for example where appropriate diluted or concentrated, and stored in the usual manner.

In one embodiment, the disease state is selected from: cancer, autoimmune disease, a developmental disorder, a genetic disorder, diabetes, cardiovascular disease, kidney disease, lung disease, liver disease, neurological disease, viral infection or bacterial infection. In a further embodiment, the disease state is cancer or autoimmune disease. In a yet further embodiment, the disease state is cancer, for example breast, bowel, bladder, bone, brain, cervical, colon, endometrial, oesophageal, kidney, liver, lung, ovarian, pancreatic, prostate, skin, stomach, testicular, thyroid or uterine cancer, leukaemia, lymphoma, myeloma or melanoma.

References herein to an "autoimmune disease" include conditions which arise from an immune response targeted against a person's own body, for example Acute disseminated encephalomyelitis (ADEM), Ankylosing Spondylitis, Behçet's disease, Celiac disease, Crohn's disease, Diabetes mellitus type 1, Graves' disease, Guillain-Barré syndrome (GBS), Psoriasis, Rheumatoid arthritis, Rheumatic fever, Sjögren's syndrome, Ulcerative colitis and Vasculitis.

References herein to a "developmental disorder" include conditions, usually originating from childhood, such as learning disabilities, communication disorders, Autism, Attention-deficit hyperactivity disorder (ADHD) and Developmental coordination disorder.

References herein to a "genetic disorder" include conditions which result from one or more abnormalities in the genome, such as Angelman syndrome, Canavan disease, Charcot-Marie-Tooth disease, Colour blindness, Cri du chat syndrome, Cystic fibrosis, Down syndrome, Duchenne muscular dystrophy, Haemochromatosis, Haemophilia, Klinefelter syndrome, Neurofibromatosis, Phenylketonuria, Polycystic kidney disease, Prader-Willi syndrome, Sickle-cell disease, Tay-Sachs disease and Turner syndrome.

Also disclosed herein is a kit for identifying a nucleic acid segment which interacts with a target nucleic acid segment or segments, which comprises buffers and reagents capable of performing the methods defined herein.

The kit may include one or more articles and/or reagents for performance of the method. For example, an oligonucleotide probe, pair of amplification primers and/or recombinase enzyme associated oligonucleotides for use in the methods described herein may be provided in isolated form and may be part of a kit, e.g. in a suitable container such as a vial in which the contents are protected from the external environment. The kit may include instructions for use according to the protocol of the method described herein. A kit wherein the nucleic acid is intended for use in PCR may include one or more other reagents required for the reaction, such as polymerase, nucleotides, buffer solution etc.

In one embodiment, the kit comprises a recombinase enzyme. In a further embodiment, the recombinase enzyme comprised in the kit as defined herein is a transposase enzyme, such as a hyperactive mutant transposases enzyme, e.g. a hyperactive mutant Tn5 transposase.

According to a yet further aspect of the invention, there is provided a recombinase enzyme as defined herein capable of single step fragmentation and adapter insertion. Thus, there is also provided herein, a recombinase enzyme capable of tagmentation.

In one embodiment, the recombinase enzyme provided herein is a hyperactive mutant transposase enzyme. In a further embodiment, the transposase enzyme is hyperactive mutant Tn5 transposase. In a yet further embodiment, the transposase enzyme comprises paired end adapter sequences.

It will be understood that examples of the types of buffers and reagents to be included in the kit, in addition to those previously described can be seen in the Examples described herein.

The following studies and protocols illustrate embodiments of the methods described herein:

### EXAMPLES

### Abbreviations:

- BB: Binding buffer
- BSA: Bovine Serum Albumin
- dd: dideoxy
- EDTA: Ethylenediaminetetraacetic acid
- HB: Agilent Hybridization buffer (HBI, HBII, HBIII and HBIV)
- NaCl: Sodium Chloride
- NTB: No Tween Buffer
- PBS: Phosphate Buffered Saline
- PCR: Polymerase Chain Reaction
- PE: Paired-end
- rpm: Revolutions Per Minute
- SDS: Sodium Dodecyl Sulphate
- SPRI beads: Solid Phase Reversible Immobilisation beads
- TB: Tween buffer
- Tn5: Transposase
- Tris-HCl: Tris(hydroxymethyl)aminomethane Hydrochloride
- WB: Wash buffer

### Cell Fixation

1. For a single experiment a minimum of 50000 cells have to be fixed for 10 minutes at room temperature at a final formaldehyde concentration of 2%.
   - Quench with glycine at the final concentration of 0.125M.
   - Centrifuge at 1500rpm (400xg) for 5 minutes at 4°C.
   - Discard supernatant, re-suspend pellet carefully in 100µl of cold 1xPBS. Centrifuge at 1500 rpm (400xg) for 5 minutes at 4°C.
   - Discard supernatant and either snap freeze in liquid nitrogen or proceed directly to the next step.

### Cell Permeabilization and Restriction Digestion

2. Resuspend the fixed cell pellet from Step 1 in 100µl of ice-cold Lysis buffer. Incubate the tube for 30 min on ice.
3. Centrifuge the tube at ~600g for 5 min at 4°C.
4. Remove the supernatant, leaving ~20µl of solution with the nuclei pellet.
5. Wash the pellet twice in 100µl 1.2x NEBuffer3 (if using Dpn II) or NEBuffer2 (if using Hind III).
6. Remove the supernatant, leaving ~20µl. Add 334µl of 1.2x NEBuffer3 (or NEBuffer2 if working with Hind III).
7. Add 12µl of 10% SDS (final concentration 0.3%, w/v); shake at 950 rpm for 1h at 37°C on a thermomixer.
8. Add 80µl of 10% Triton (final concentration 1.8%, v/v); shake at 950 rpm for 1h at 37°C on a thermomixer.
9. Add 30µl of Dpn II (50 U/µl) (or 15µl of Hind III - 100U/µl and 15µl of H₂O) and shake at 950 rpm at 37°C on a thermomixer for 12-16h.

### Biotin Labelling and Hi-C Ligation

10. Briefly spin the digestion mix.
11. Add 4.5µl of dCTP, dTTP and dGTP (10mM mix), 37.5µl of biotin-dATP and 10µl of Klenow (5 U/µl). Incubate at for 45 min at 37°C shaking at 700 rpm for 10s every 30s.
12. Centrifuge at 600g for 6min at 4°C.
13. Remove the supernatant, leaving ~50µl including the pellet.
14. Add 835µl of H₂O / 100µl T4 DNA ligase buffer / 5µl BSA (20 mg/ml) / 10µl T4 DNA ligase (Invitrogen).
15. Incubate for 4h minimum (or up to 12 hours) at 16°C.

### Purification of Hi-C DNA

16. Centrifuge the tube at 600g, 4°C for 6 min.
17. Remove 800µl of supernatant, leaving 200µl in the tube.
18. Add 15µl of proteinase K (10 mg/ml). Incubate at 65°C for 4h (optional).
19. Add 15µl of proteinase K (10 mg/ml). Incubate at 65°C for o/n.
20. Purify with 1x volume of SPRI beads (Beckman Coulter Ampure XP beads A63881), following the manufacturer's instructions. Do not overdry the beads, as this may decrease the recovery of long DNA fragments. Incubate in nuclease free water for 10 min.

### Tagmentation

21. Set up several tagmentation reactions (according to the total amounts of collected DNA) as follows:
   - X µl of DNA
   - 4µl of Tagmentation buffer (5X)
   - Y µl of Tn5
   - 16 - X -Y of nuclease free water
      Incubate for 7 min at 55°C without mixing.
      Aim for DNA fragment distribution around 400 bp.
      As a guideline: use 0.5-1 µl of 12.3uM Tn5 if working with ~50ng of DNA. If working with 100-300ng of DNA, use 1µl of 24.6uM Tn5.
      For better results - titrate the amount of Tn5 to get a proper fragment distribution.
22. Check the DNA fragment distribution on TapeStation or Bioanalyzer:
   - Use 1µl from the tagmentation mix, add 3µl of H₂O and 1µl of 0.2% SDS. Incubate for 7 min at 55°C.
   - Strip off the Transposase by adding 5µl of 0.2% SDS and incubating at 55°C for 7 min.
   - Use 2µl from this mix to load on TapeStation or Bioanalyzer.
   If the distribution is correct - add 1µl of nuclease free water to the initial tagmentation mix from step 21 and strip off the Tn5 by adding 5µl of 0.2% SDS and incubating at 55°C for 7 min.
23. Combine 25µl of this tagmentation mix with the leftover 3µl of the mix from step 22.

### Pull Down of Hi-C Ligation Products

24. Use 25µl of Streptavidin MyOne C1 Dynabeads per sample for a pull down of ligation events. To prepare beads wash them twice with 400µl of TB buffer (3 min rotation per wash). Resuspend the 25µl of beads in 50µl of 2xNTB buffer.
25. Mix together the beads (from the previous step) with 22µl of TLE and 28µl of the tagmentation mix (from the step 24). Incubate at RT for 45 min, rotating slowly.
26. Wash the beads four times with 100µl of 1xNTB, followed by two washes with 50µl of TLE. Resuspend the beads in 25µl of nuclease-free water.

### Library Preparation

27. Make 5 reaction as follows:
- 5µl from the previous mix
- 29.5µl of H₂O
- 10µl of KAPA HiFi buffer (5x)
- 1.5µl dNTPs (10mM)
- 1µl KAPA HiFi DNA polymerase
- 3µl i7/i5 primers (10uM) mix

### PCR conditions:

- 3' at 72°C
- 4-7 cycles of {10" 95°C, 30" 55°C, 30", 72°C}
- 5' 72°C

28. Combine reactions and purify with Ampure SPRI beads (1x ratio). Check the quality and quantity of the Capture Hi-C library by TapeStation/Bioanalyzer and Qubit.

### Capture Hybridization of Hi-C Library with Biotin-RNA - Method 1

### Prepare three PCR strips: "DNA", "Hybridization" and "RNA".

29a. Prepare Hi-C library: transfer volume equivalent to between 300ng and 1µg, in particular 500ng, of Hi-C library into a 1.5ml Eppendorf tube and dry using a SpeedVac (45°C, ~15 minutes). Resuspend the Hi-C DNA pellet in 4µl of nuclease free water.

30a. Prepare blockers mix. Per sample:
- blocker # 1 - 2.5µl (Agilent Technologies)
- blocker # 2 - 2.5µl (Agilent Technologies)
- custom blockers - 1µl

31a. Mix blockers mix from the previous step with the DNA library from the step 29. Transfer 10µl of the DNA library into the well of the corresponding PCR strip. Keep on ice.

32a. Prepare a hybrid mix. Keep it at RT.
- HBI - 25µl
- HBII - 1µl
- HBIII - 10µl
- HBIV - 13µl

Mix thoroughly; if a precipitate has formed, heat at 65°C for 5 minutes. Aliquot 30µl per capture to each well in "Hybridization" PCR strip (Agilent 410022), close with a PCR strip tube lid (Agilent optical cap 8x strip 401425) and keep at room temperature.

33a. Prepare RNase block solution 1:4 (e.g. 3µl RNase block + 9µl water).

34a. Prepare biotin-RNA. Per capture: mix 5µl of custom baits (or 2ul custom baits + 3ul of nuclease-free water, if the capture system size is <3Mb) with 2µl of RNase block dilution. Transfer these 7µl to the "RNA" PCR strip. Keep on ice.

35a. Hybridization reaction: Set the PCR thermocycler to the following program: 95°C for 5', 65°C - ∞

*The PCR machine lid has to be heated. Throughout the procedure, work quickly and try to keep the PCR machine lid open for the minimum time possible. Evaporation of the sample will result in suboptimal hybridization conditions.*

36a. Transfer the "DNA" PCR strip with the Hi-C library to the PCR machine, in the position marked in black below, and start the PCR program. Incubate DNA for 5 min at 95°C.

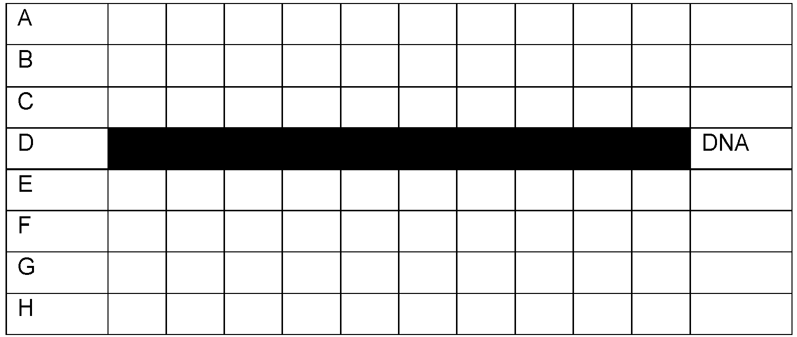

37a. Once the temperature has reached 65°C, transfer the "Hybridization" PCR strip with the hybridization buffer to the PCR machine, in the position marked in grey below. Incubate at 65°C for 5 mins.

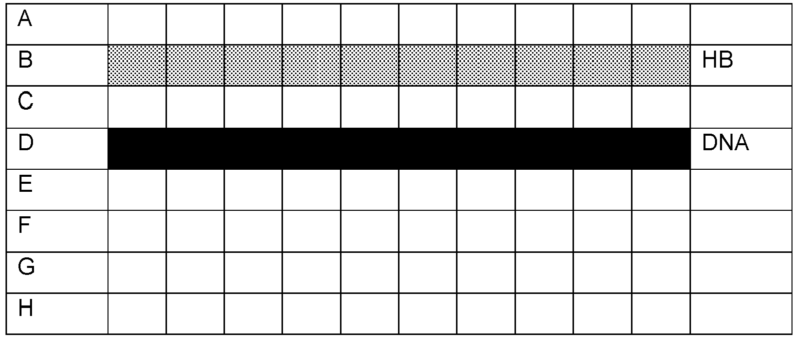

38a. Transfer the "RNA" PCR strip with the biotinylated RNA bait to the PCR machine, in the position marked in cross-hatching below. Incubate for 2 mins.

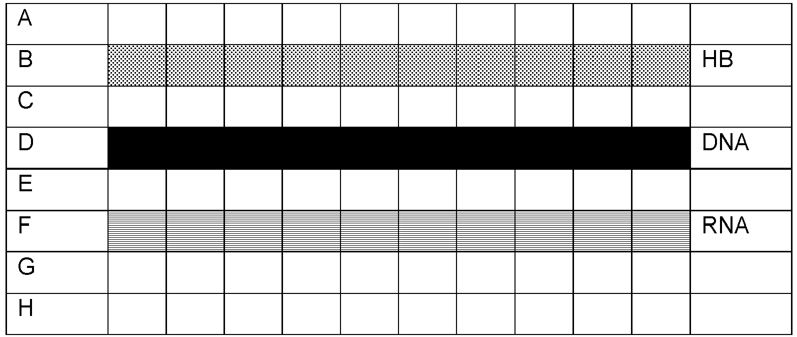

39a. Open "Hybridization" and "RNA" strips. Pipette 13µl of hybridization buffer into the 7µl of RNA bait (grey into cross-hatched). Discard the PCR strip containing the hybridization buffer. Proceed immediately to the next step.

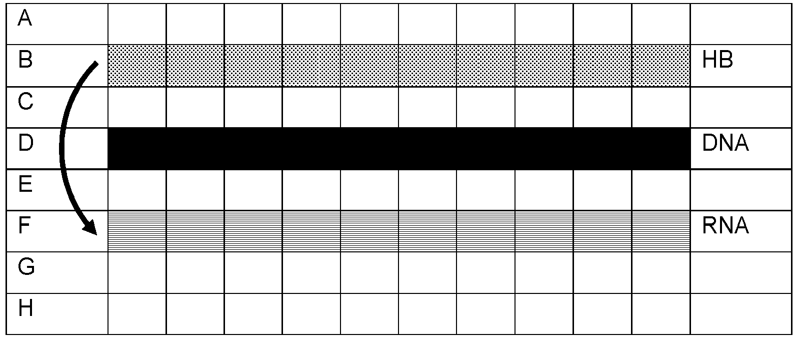

40a. Take off the lid from the "DNA" PCR strip containing the Hi-C library. Pipette 10µl of the Hi-C library into the 20µl of RNA bait with hybridization buffer (black into cross-hatched). Check that nothing is left in the DNA PCR strip and discard it.

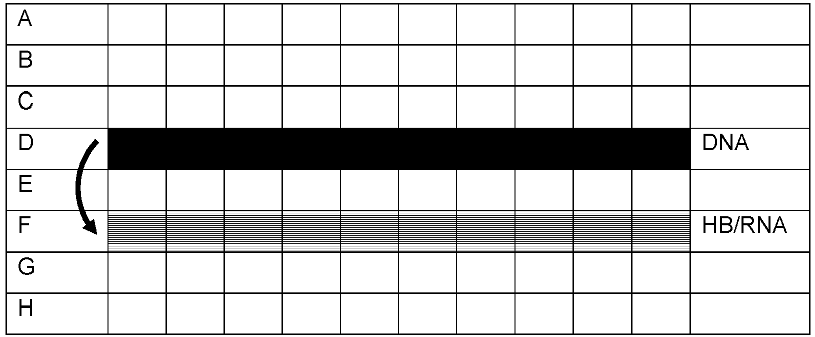

Close the remaining "RNA" PCR strip (now containing Hi-C library/hybridization buffer/RNA bait) with a fresh PCR strip tube lid immediately and incubate for 24 hours at 65°C.

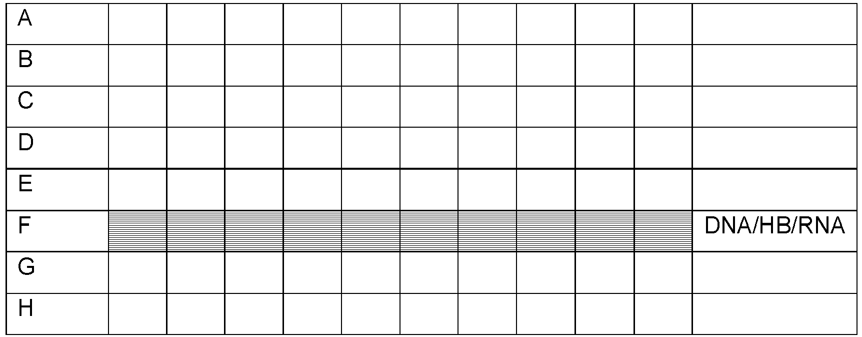

### Streptavidin-Biotin Pull-Down and Washes - to be used with Method 1 above

41a. Prepare buffers:
Binding buffer (BB, Agilent Technologies) at room temperature
Wash buffer I (WB I, Agilent Technologies) at room temperature
Wash buffer II (WB II Agilent Technologies) at between 65°C and 72°C, in particular at 65°C NEB2 1x (NEB B7002S) at room temperature.

42a. Wash magnetic beads:
Mix Dynabeads MyOne Streptavidin T1 (Life Technologies 65601) thoroughly before adding 60µl per Capture Hi-C sample into a 1.5 ml lobind Eppendorf tube. Wash the beads as follows (same procedures for all subsequent wash steps):
- Add 200µl BB
- Mix on vortex (at low to medium setting) for 5 seconds.
- Place tube on Dynal magnetic separator (Life Technologies)
- Reclaim beads, discard supernatant

Repeat steps a) to d) for a total of 3 washes.

43a. Biotin-Streptavidin pulldown:
With the Dynabeads MyOne Streptavidin T1 beads in 200µl BB in a fresh low bind Eppendorf tube, open the lid of the PCR machine (while the PCR machine is running) and pipette the entire hybridization reaction into the tube containing the streptavidin beads. Incubate on a rotating wheel for 30 mins at room temperature.

44a. Washes:
After 30 mins, place the sample on the magnetic separator, discard supernatant.

Resuspend beads in 500µl WB I, and transfer to a fresh tube. Incubate at room temperature for 15 mins. Vortex every 2 to 3 minutes for 5 seconds each.

Separate the beads and buffer on a magnetic separator and remove the supernatant. Resuspend in 500µl WB II (prewarmed to between 65°C and 72°C, in particular 65°C) and transfer to a fresh tube. Incubate at between 65°C and 72°C, in particular at 65°C, for 10 mins, and vortex (at low to medium setting) for 5 seconds every 2 to 3 minutes. Repeat for a total of 3 washes in WB II, all at between 65°C and 72°C, in particular at 65°C.

Resuspend in 200µl of Neb2 1X. Put directly on the magnet. Remove the supernatant and resuspend in 30µl of Neb2 1X.

The RNA/DNA mixture hybrid 'catch' on beads is now ready for PCR amplification (step 45).

### Capture Hybridization of Hi-C Library with Biotin-RNA - Method 2 (using Buffers with High Concentrations of Divalent Cation Salt- herein referred to as "Fast Hybridization")

As described herein, according to embodiments utilising this method, preparation time may be greatly reduced (for example, to approx. 2 hours 45 minutes).
29b. Pre-warm fast hybridization buffer at room temperature until thawed and keep at room temperature until ready to use
30b. Prepare blocker mix:
   - 2.5µl of 1mg/ml Cot-1 DNA from the same species from which the nucleic acid composition is derived, such as human Cot-1 DNA
   - 2.5µl of 10mg/ml salmon sperm DNA
   - 1µl custom blockers
31b. Set up blocking reactions at room temperature as following:
   - Add 6µl of a blocker mix prepared above to 11µl prepared DNA sample (approx. 100ng-1µg, e.g. 500ng)
   - Pipet up and down to mix. Spin down briefly
32b. Program a thermal cycler as shown below. Start the program and hit the pause button immediately. This will heat the lid while adding the blocker mix to a pre-prepared library of genomic DNA fragments.
   - Denaturation - 95°C for 5 minutes
   - Blocking - 65°C for 10 minutes
   - Hybridization - 50 cycles - 65°C for 1 minute; 37°C for 3 seconds
   - Storage - 65°C hold
33b. Put the sample into the thermal cycler and resume the program to perform denaturation and blocking.
34b. While the samples are incubating on the thermal cycler, prepare the capture bait mix on ice.
35b. Dilute a SureSelect RNase Block for capture (1 part RNase Block: 3 parts water):
   - Mix 1µl of the RNase Block (Agilent Technologies Inc.)
   - 3µl of water
36b. Prepare the hybridization mix:
   - 2µl diluted SureSelect RNase block
   - 5µl SureSelect custom baits (or 2ul SureSelect custom baits + 3ul of nuclease-free water, if the capture system is <3Mb)
   - 6µl room temperature 5X fast hybridization buffer
37b. When the thermal cycle reaches the first hybridization cycle at 65°C, hit the pause button. The thermal cycler is now maintaining at 65°C. Open the thermal cycler lid and pipet 13µl of the hybridization mix into each corresponding blocking reactions. Mix well by slowly pipetting up and down 8 to 10 times. The hybridization reaction is now 30µl.
38b. Seal the wells with caps, close the lid, and hit the play button to resume the program to run the cycling hybridization profile with the heated lid activated.
39b. Prepare magnetic beads (Dynabeads MyOne Streptavidin T1, Invitrogen)
   - Vigorously resuspend the Dynal (Invitrogen) magnetic beads on a vortex mixer
   - For each hybridization sample use 60µl Dynabeads T1 Magnetic beads
   - Wash the beads:
      (a) Add 200µl SureSelect Binding buffer (Agilent Technologies Inc.)
      (b) Mix the beads by pipetting up and down 10 times
      (c) Put tubes on a magnetic stand
      (d) Wait for 2-5 minutes and discard the supernatant
      (e) Repeat step (a) through step (d) for a total of 3 washes
      (f) Resuspend the beads in 20µl of SureSelect Binding buffer
40b. Capture the hybridized DNA using streptavidin beads
   - After the incubation remove the samples from the thermal cycler and briefly spin at room temperature to collect the liquid
   - Add the entire hybridization mixture for each sample to the corresponding washed and ready Dynal MyOne T1 Streptavidin beads solution and invert the strip-tubes/plate to mix 3 to 5 times
   - Incubate the hybrid-capture/bead solution on a rotator or shaker for 30 minutes at room temperature
   - Pre-warm wash buffer #2 at between 68°C and 72°C, in particular at 68°C, by aliquoting out 1500µl per sample
   - Briefly spin down the hybrid-capture/bead solution after 30 minutes
41b. Wash the beads:
   (a) Separate the beads and buffer on a magnetic separator and remove the supernatant
   (b) Resuspend the beads in 500µl wash buffer #1 by pipetting up and down 8-10 times then leave for 10 minutes at 23°C. Separate the beads and buffer on a magnetic separator and remove the supernatant
   (c) Repeat steps (a) to (b)
   (d) Separate the beads and buffer on a magnetic stand for 1 minute and remove the supernatant
   (e) Add 500µl of pre-warmed wash buffer #2. Slowly pipette up and down 10 times to resuspend the beads. When pipetting the wash buffer up and down dispense the buffer directly at the pelleted beads to resuspend them faster
   (f) Incubate the samples for 10 minutes at between 68°C and 72°C, in particular at 68°C
   (g) Repeat steps (d) through step (f) for a total of 3 washes
   (h) Separate the beads and buffer on a magnetic stand. Make sure the entire wash buffer #2 has been removed
   (i) Resuspend in 50µl of nuclease free water, separate the beads on a magnetic stand and remove the supernatant
   (j) Resuspend the beads in 23µl of nuclease free water, and proceed to the PCR.

Proceed to PCR amplification of capture Hi-C library (step 45).

### PCR Amplification of Capture Hi-C Library

45. Set up PCRs with 5 amplification cycles as following:
- 5µl from the previous mix
- 29.5µl of mQ (water)
- 10µl of KAPA HiFi buffer (5x)
- 1.5µl dNTPs (10mM)
- 1µl KAPA HiFi DNA polymerase
- 3µl primers (10uM) mix (P5-FCA-R and FCA-P7F)

PCR conditions:
- 3' at 95°C
- 5 cycles of {20" 95°C, 30" 55°C, 30", 72°C}
- 3' 72°C

46. Pool all individual PCR reactions from the step above. Place on a magnetic separator and transfer the supernatant into a fresh 1.5ml lobind Eppendorf tube. Purify with 1X volume of SPRI beads (Beckman Coulter Ampure XP beads A63881), following the manufacturer's instructions. Resuspend in a final volume of 20µl TLE or nuclease free water.

Check the quality and quantity of the Capture Hi-C library by TapeStation/Bioanalyzer and KAPA qPCR.

### Tn5 Transposase Adapter Sequences

Sequences used for the assembly on the Tn5 transposase:

| | | |
|---|---|---|
| Tn5MErev | 5'-[phos]**CTGTCTCTTATACACATCT**-3' | SEQ ID NO: 1 |
| FC-A | 5'-GTCTCGTGGGCTCGG**AGATGTGTATAAGAGACAG**-3' | SEQ ID NO: 2 |
| FC-B | 5'-TCGTCGGCAGCGTC**AGATGTGTATAAGAGACAG**-3' | SEQ ID NO: 3 |

### Primers for Pre-Capture PCR

| | | |
|---|---|---|
| Dual-i7-rcN701 | CAAGCAGAAGACGGCATACGAGATTAAGGCGAGTC TCGTGGGCTCGG | SEQ ID NO: 4 |
| Dual-i7-rcN702 | CAAGCAGAAGACGGCATACGAGATCGTACTAGGTC TCGTGGGCTCGG | SEQ ID NO: 5 |
| Dual-i7-rcN705 | CAAGCAGAAGACGGCATACGAGATGGACTCCTGTC TCGTGGGCTCGG | SEQ ID NO: 6 |
| Dual-i7-rcN706 | CAAGCAGAAGACGGCATACGAGATTAGGCATGGTC TCGTGGGCTCGG | SEQ ID NO: 7 |
| Dual-i5-S503 | AATGATACGGCGACCACCGAGATCTACACTATCCT CTTCGTCGGCAGCGTC | SEQ ID NO: 8 |
| Dual-i5-S504 | AATGATACGGCGACCACCGAGATCTACACAGAGTA GATCGTCGGCAGCGTC | SEQ ID NO: 9 |

| | | |
|---|---|---|
| "rc" denotes that barcode sequences are reverse-complemented. | | |

### Blocker Sequences

| | | |
|---|---|---|
| i5Rdd | TCGTCGGCAGCGTCAGATGTGTATAAGAGA/3ddC/ | SEQ ID NO: 10 |
| ddi7F | GTCTCGTGGGCTCGGAGATGTGTATAAGAGA/3ddC/ | SEQ ID NO: 11 |
| i5F | CTGTCTCTTATACACATCTGACGCTGCCGACGA | SEQ ID NO: 12 |
| P5-FCA-F | GTGTAGATCTCGGTGGTCGCCGTATCATT | SEQ ID NO: 13 |
| P5-FCA-R | AATGATACGGCGACCACCGAGATCTACAC | SEQ ID NO: 14 |
| i7R | CTGTCTCTTATACACATCTCCGAGCCCACGAGAC | SEQ ID NO: 15 |
| FCA-P7F | CAAGCAGAAGACGGCATACGAGAT | SEQ ID NO: 16 |
| FCA-P7R | ATCTCGTATGCCGTCTTCTGCTTG | SEQ ID NO: 17 |

### Buffer Solutions

### 5X Fast Hybridization buffer

1540 mM MgCl₂*6H₂O, 0.0417% w/w HPMC, 100 mM Tris (pH 8.0) and H₂O.

### Wash buffer #1

("low-stringency buffer" - high salt concentrations and low temperatures, to remove non-specifically bound probe) 2X SSC, 0.1% SDS and H₂O.

### Wash buffer #2

("high-stringency buffer" - low salt concentrations and high temperatures, to remove low-affinity hybridization probe) 0.1X SSC, 0.1% SDS and H₂O.

## Claims

1. A method for identifying nucleic acid segments which interact with a target nucleic acid segment or segments, said method comprising the steps of:
(a) obtaining a nucleic acid composition comprising the target nucleic acid segment or segments;
(b) crosslinking the nucleic acid composition;
(c) fragmenting the crosslinked nucleic acid composition using an endonuclease enzyme;
(d) filling the ends of the fragmented crosslinked nucleic acid segments with one or more nucleotides comprising a covalently linked biotin moiety;
(e) ligating the fragmented nucleic acid segments obtained from step (d) to produce ligated fragments;
(f) performing single step fragmentation and oligonucleotide insertion on the ligated fragments using a recombinase enzyme;
(g) enriching for fragments comprising the biotin moiety of step (d);
(h) enriching fragments comprising the target nucleic acid segment or segments in the presence of sequences which prevent the binding of ligated fragments to other ligated fragments through complementarity of adapter sequences, such as blocker sequences;
(i) sequencing the enriched fragments obtained in step (h) to identify the nucleic acid segments which interact with the target nucleic acid segment or segments.

2. The method of claim 1, wherein step (h) comprises:
(i) performing targeted amplification to enrich fragments comprising the target nucleic acid segment or segments; or
(i) addition of isolating nucleic acid molecules which bind to the target nucleic acid segment or segments, wherein said isolating nucleic acid molecules are labelled with a first half of a binding pair; and
(ii) isolating fragments which contain the target nucleic acid segment or segments bound to the isolating nucleic acid molecules by using the second half of said binding pair.

3. The method of claim 1 or claim 2, wherein step (f) is performed by tagmentation; and/or step (e) utilises in-nucleus ligation.

4. The method of any one of claims 1 to 3, wherein the recombinase enzyme:
(i) is a retroviral integrase, such as a mutant transposase, such as hyperactive Tn5 transposase; and/or
(ii) comprises paired end adapter sequences for sequencing or fragments thereof.

5. The method of any one of claims 1 to 4, wherein the oligonucleotide and/or adapter sequences comprise a barcode sequence.

6. The method of claim 4 or claim 5, wherein said oligonucleotide and/or adapter sequences are selected from: SEQ ID NO: 1, SEQ ID NO: 2 and/or SEQ ID NO: 3, or oligonucleotide sequences that enable subsequent library preparation and sequencing.

7. The method of any one of claims 1 to 6, wherein the target nucleic acid segment or segments is selected from: promoters, silencers, enhancers or insulators.

8. The method of any one of claims 1 to 7, wherein the isolating nucleic acid molecules are:
(i) obtained from bacterial artificial chromosomes (BACs), fosmids or cosmids; and/or
(ii) RNA.

9. The method of any one of claims 2 to 8, wherein the first half of the binding pair comprises biotin and the second half of the binding pair comprises streptavidin.

10. The method of any one of claims 1 to 9, wherein the restriction enzyme used at step (c) is Hind III or Dpn II.

11. The method of any one of claims 2 to 10, which additionally comprises at step (g) amplifying the enriched fragments comprising the biotin moiety.

12. The method of any one of claims 1 to 11, which additionally comprises amplifying the isolated ligated fragments prior to step (i); and/or wherein the targeted amplification or amplifying is performed by PCR.

13. The method of any one of claims 1 to 12, wherein said nucleic acid composition is derived from:
(i) a mammalian cell nucleus, such as a human cell nucleus; and/or
(ii) a non-human cell nucleus, such as a mouse cell nucleus or plant cell nucleus; and/or
(iii) 10000, 50000, 0.2 million, 0.5 million or 1 million cells.

14. A method of identifying one or more interacting nucleic acid segments that are indicative of a particular disease state, such as cancer, an autoimmune disease, a developmental disease or a genetic disorder, comprising:
(a) performing the method of any one of claims 1 to 13 on a nucleic acid composition obtained from an individual with a particular disease;
(b) quantifying a frequency of interaction between a nucleic acid segment and a target nucleic acid segment or segments; and
(c) comparing the frequency of interaction in the nucleic acid composition from the individual with said disease state with the frequency of interaction in a normal control nuclear composition from a healthy subject, such that a difference in the frequency of interaction in the nucleic acid composition is indicative of a particular disease.

15. A kit for identifying a nucleic acid segment which interacts with a target nucleic acid segment or segments, comprising buffers and reagents suitable for performing the steps of the method of any one of claims 1 to 13 in combination with sequences which prevent the binding of ligated fragments to other ligated fragments through complementarity of adapter sequences, such as blocker sequences, optionally wherein the recombinase enzyme is a retroviral integrase or a transposase enzyme, such as a mutant transposase enzyme, such as hyperactive Tn5 transposase.

## Patentansprüche

1. Verfahren zum Identifizieren von Nukleinsäuresegmenten, die mit einem oder mehreren Zielnukleinsäuresegmenten interagieren, wobei das Verfahren die folgenden Schritte umfasst:
(a) Erlangen einer Nukleinsäurezusammensetzung, die das oder die Zielnukleinsäuresegmente umfasst;
(b) Vernetzen der Nukleinsäurezusammensetzung;
(c) Fragmentieren der vernetzten Nukleinsäurezusammensetzung unter Verwendung eines Endonuklease-Enzyms;
(d) Füllen der Enden der fragmentierten, vernetzten Nukleinsäuresegmente mit einem oder mehreren Nukleotiden, die eine kovalent verknüpfte Biotineinheit umfassen;
(e) Ligieren der aus Schritt (d) erlangten fragmentierten Nukleinsäuresegmente zum Herstellen ligierter Fragmente;
(f) Durchführen einer Einstufen-Fragmentierung und Oligonukleotidinsertion an den ligierten Fragmenten unter Verwendung eines Rekombinase-Enzyms;
(g) Anreichern von Fragmenten, die die Biotineinheit aus Schritt (d) umfassen;
(h) Anreichern von Fragmenten, die das oder die Zielnukleinsäuresegmente umfassen, in Gegenwart von Sequenzen, die die Bindung ligierter Fragmente an andere ligierte Fragmente durch Komplementarität von Adaptersequenzen, wie etwa Blockersequenzen, verhindern;
(i) Sequenzieren der in Schritt (h) erlangten angereicherten Fragmente zum Identifizieren der Nukleinsäuresegmente, die mit dem oder den Zielnukleinsäuresegmenten interagieren.

2. Verfahren nach Anspruch 1, wobei Schritt (h) umfasst:
(i) Durchführen einer gezielten Amplifikation zum Anreichern von Fragmenten, die das oder die Zielnukleinsäuresegmente umfassen; oder
(i) Zusetzen von isolierenden Nukleinsäuremolekülen, die an das oder die Zielnukleinsäuresegmente binden, wobei die isolierenden Nukleinsäuremoleküle mit einer ersten Hälfte eines Bindungspaares markiert sind; und
(ii) Isolieren von Fragmenten, die das oder die an die isolierenden Nukleinsäuremoleküle gebundenen Zielnukleinsäuresegmente enthalten, unter Verwendung der zweiten Hälfte des Bindungspaares.

3. Verfahren nach Anspruch 1 oder Anspruch 2, wobei Schritt (f) durch Tagmentation durchgeführt wird; und/oder Schritt (e) eine kerninterne Ligation verwendet.

4. Verfahren nach einem der Ansprüche 1 bis 3, wobei das Rekombinase-Enzym:
(i) eine retrovirale Integrase ist, wie etwa eine mutierte Transposase, wie etwa hyperaktive Tn5-Transposase; und/oder
(ii) Adaptersequenzen mit gepaarten Enden zum Sequenzieren oder Fragmente davon umfasst.

5. Verfahren nach einem der Ansprüche 1 bis 4, wobei die Oligonukleotid- und/oder Adaptersequenzen eine Barcodesequenz umfassen.

6. Verfahren nach Anspruch 4 oder Anspruch 5, wobei die Oligonukleotid- und/oder Adaptersequenzen ausgewählt sind aus: SEQ ID NO: 1, SEQ ID NO: 2 und/oder SEQ ID NO: 3, oder Oligonukleotidsequenzen, die eine nachfolgende Bibliothekspräparation und -sequenzierung ermöglichen.

7. Verfahren nach einem der Ansprüche 1 bis 6, wobei das oder die Zielnukleinsäuresegmente ausgewählt ist/sind aus: Promotoren, Silencern, Enhancern oder Isolatoren.

8. Verfahren nach einem der Ansprüche 1 bis 7, wobei die isolierenden Nukleinsäuremoleküle Folgendes sind:
(i) erlangt aus künstlichen Bakterienchromosomen (BAC), Fosmiden oder Cosmiden und/oder
(ii) RNA.

9. Verfahren nach einem der Ansprüche 2 bis 8, wobei die erste Hälfte des Bindungspaares Biotin umfasst und die zweite Hälfte des Bindungspaares Streptavidin umfasst.

10. Verfahren nach einem der Ansprüche 1 bis 9, wobei das in Schritt (c) verwendete Restriktionsenzym Hind III oder Dpn II ist.

11. Verfahren nach einem der Ansprüche 2 bis 10, das zusätzlich in Schritt (g) Amplifizieren der angereicherten Fragmente, die die Biotineinheit umfassen, umfasst.

12. Verfahren nach einem der Ansprüche 1 bis 11, das zusätzlich Amplifizieren der isolierten ligierten Fragmente vor Schritt (i) umfasst; und/oder wobei die gezielte Amplifikation oder Amplifizieren mittels PCR durchgeführt wird.

13. Verfahren nach einem der Ansprüche 1 bis 12, wobei die Nukleinsäurezusammensetzung abgeleitet ist von:
(i) einem Zellkern von Säugern, wie etwa einem Zellkern von Menschen; und/oder
(ii) einem nicht-menschlichen Zellkern, wie etwa einem Maus- oder Pflanzenzellkern; und/oder
(iii) 10000, 50000, 0,2 Millionen, 0,5 Millionen oder 1 Million Zellen.

14. Verfahren zum Identifizieren eines oder mehrerer interagierender Nukleinsäuresegmente, die auf einen bestimmten Krankheitszustand hinweisen, wie etwa Krebs, eine Autoimmunkrankheit, eine Entwicklungskrankheit oder eine genetische Störung, umfassend:
(a) Durchführen des Verfahrens nach einem der Ansprüche 1 bis 13 an einer Nukleinsäurezusammensetzung, die von einem Individuum mit einer bestimmten Krankheit erhalten wurde;
(b) Quantifizieren einer Interaktionshäufigkeit zwischen einem Nukleinsäuresegment und einem oder mehreren Zielnukleinsäuresegmenten; und
(c) Vergleichen der Interaktionshäufigkeit bei der Nukleinsäurezusammensetzung von dem Individuum mit dem Krankheitszustand mit der Interaktionshäufigkeit bei einer normalen Kontroll-Kernzusammensetzung von einem gesunden Subjekt, sodass ein Unterschied in der Interaktionshäufigkeit bei der Nukleinsäurezusammensetzung auf eine bestimmte Krankheit hinweist.

15. Kit zum Identifizieren eines Nukleinsäuresegments, das mit einem oder mehreren Zielnukleinsäuresegmenten interagiert, umfassend Puffer und Reagenzien, die zum Durchführen der Schritte des Verfahrens nach einem der Ansprüche 1 bis 13 in Kombination mit Sequenzen geeignet sind, die die Bindung von ligierten Fragmenten an andere ligierte Fragmente durch Komplementarität von Adaptersequenzen, wie etwa Blockersequenzen, verhindern, wobei optional das Rekombinaseenzym eine retrovirale Integrase oder ein Transposase-Enzym, wie etwa ein mutiertes Transposase-Enzym, wie etwa hyperaktive Tn5-Transposase, ist.

## Revendications

1. Procédé d'identification de segments d'acide nucléique interagissant avec un ou des segments d'acide nucléique cibles, ledit procédé comprenant les étapes suivantes :
(a) l'obtention d'une composition d'acide nucléique comprenant le ou les segments d'acide nucléique cibles ;
(b) la réticulation de la composition d'acide nucléique ;
(c) la fragmentation de la composition d'acide nucléique réticulée à l'aide d'une enzyme endonucléase ;
(d) le remplissage des extrémités des segments d'acide nucléique réticulés fragmentés avec un ou plusieurs nucléotides comprenant une fraction de biotine liée de manière covalente ;
(e) la ligature des segments d'acide nucléique fragmentés obtenus à l'étape (d) pour produire des fragments ligaturés ;
(f) la réalisation d'une fragmentation en une seule étape et l'insertion d'oligonucléotides sur les fragments ligaturés à l'aide d'une enzyme recombinase ;
(g) l'enrichissement de fragments comprenant la fraction biotine de l'étape (d) ;
(h) l'enrichissement de fragments comprenant le ou les segments d'acide nucléique cibles en présence de séquences qui empêchent la liaison de fragments ligaturés à d'autres fragments ligaturés par complémentarité de séquences d'adaptateurs, telles que des séquences de bloqueurs ;
(i) le séquençage des fragments enrichis obtenus à l'étape (h) pour identifier les segments d'acide nucléique qui interagissent avec le ou les segments d'acide nucléique cibles.

2. Procédé selon la revendication 1, dans lequel l'étape (h) comprend :
(i) la réalisation d'une amplification ciblée pour enrichir des fragments comprenant le ou les segments d'acide nucléique cibles ; ou
(i) l'ajout de molécules d'acide nucléique d'isolement qui se lient au ou aux segments d'acide nucléique cibles, dans lequel lesdites molécules d'acide nucléique d'isolement sont marquées avec une première moitié d'une paire de liaison ; et
(ii) l'isolement de fragments qui contiennent le ou les segments d'acide nucléique cibles liés aux molécules d'acide nucléique isolantes en utilisant la seconde moitié de ladite paire de liaison.

3. Procédé selon la revendication 1 ou la revendication 2, dans lequel l'étape (f) est réalisée par tagmentation ; et/ou l'étape (e) utilise une ligature intra-noyau.

4. Procédé selon l'une quelconque des revendications 1 à 3, dans lequel l'enzyme recombinase :
(i) est une intégrase rétrovirale, telle qu'une transposase mutante, telle que la transposase Tn5 hyperactive ; et/ou
(ii) comprend des séquences d'adaptateurs d'extrémité appariées pour le séquençage ou des fragments de celles-ci.

5. Procédé selon l'une quelconque des revendications 1 à 4, dans lequel les séquences d'oligonucléotidiques et/ou d'adaptateurs comprennent une séquence code-barres.

6. Procédé selon la revendication 4 ou la revendication 5, dans lequel lesdites séquences d'oligonucléotides et/ou d'adaptateurs sont choisies parmi : SEQ ID NO: 1, SEQ ID NO: 2 et/ou SEQ ID NO: 3, ou des séquences d'oligonucléotides permettant la préparation et le séquençage ultérieurs de banques.

7. Procédé selon l'une quelconque des revendications 1 à 6, dans lequel le ou les segments d'acide nucléique cibles sont choisis parmi : des promoteurs, des silencieux, des renforçants ou des isolants.

8. Procédé selon l'une quelconque des revendications 1 à 7, dans lequel les molécules d'acide nucléique isolantes sont :
(i) obtenues à partir de chromosomes bactériens artificiels (BAC), de fosmides ou de cosmides ; et/ou
(ii) de l'ARN.

9. Procédé selon l'une quelconque des revendications 2 à 8, dans lequel la première moitié de la paire de liaison comprend de la biotine et la seconde moitié de la paire de liaison comprend de la streptavidine.

10. Procédé selon l'une quelconque des revendications 1 à 9, dans lequel l'enzyme de restriction utilisée à l'étape (c) est Hind III ou Dpn II.

11. Procédé selon l'une quelconque des revendications 2 à 10, qui comprend en outre à l'étape (g) l'amplification des fragments enrichis comprenant la fraction biotine.

12. Procédé selon l'une quelconque des revendications 1 à 11, qui comprend en outre l'amplification des fragments ligaturés isolés avant l'étape (i) ; dans lequel l'amplification ou les amplifications ciblées sont réalisées par PCR.

13. Procédé selon l'une quelconque des revendications 1 à 12, dans lequel ladite composition d'acide nucléique est dérivée :
(i) d'un noyau de cellule de mammifère, tel qu'un noyau de cellule humaine ; et/ou
(ii) d'un noyau de cellules non humaines, tel qu'un noyau de cellules de souris ou de cellules végétales ; et/ou
(iii) de 10 000, 50 000, 0,2 million, 0,5 million ou 1 million de cellules.

14. Procédé d'identification d'un ou de plusieurs segments d'acide nucléique interagissant indiquant un état pathologique particulier, tel que le cancer, une maladie auto-immune, une maladie développementale ou un trouble génétique, comprenant :
(a) la mise en œuvre du procédé selon l'une quelconque des revendications 1 à 13 sur une composition d'acides nucléiques obtenue à partir d'un individu atteint d'une maladie particulière ;
(b) la quantification d'une fréquence d'interaction entre un segment d'acide nucléique et un ou des segments d'acide nucléique cibles ; et
(c) la comparaison de la fréquence d'interaction dans la composition d'acide nucléique provenant de l'individu présentant ledit état pathologique avec la fréquence d'interaction dans une composition nucléaire témoin normale provenant d'un sujet sain, de sorte qu'une différence dans la fréquence d'interaction dans la composition d'acide nucléique indique une maladie particulière.

15. Kit d'identification d'un segment d'acide nucléique interagissant avec un ou des segments d'acide nucléique cibles, comprenant des tampons et des réactifs adaptés pour mettre en œuvre les étapes du procédé de l'une quelconque des revendications 1 à 13 en combinaison avec des séquences qui empêchent la liaison de fragments ligaturés à d'autres fragments ligaturés par complémentarité de séquences d'adaptateurs, telles que des séquences de bloqueurs, éventuellement dans lequel l'enzyme recombinase est une intégrase rétrovirale ou une enzyme transposase, telle qu'une enzyme transposase mutante, telle que la transposase Tn5 hyperactive.
